# EUROPEAN PATENT APPLICATION

(11) **EP 4 537 756 A1**
(43) Date of publication of application: **16.04.2025**
(21) Application number: 23818852.8
(22) Date of filing: 28.04.2023
(51) Int. Cl.: A61B 5/366, A61B 5/358

(54) **MOTION ELECTROCARDIOGRAM DATA ANALYSIS METHOD AND APPARATUS, COMPUTER DEVICE AND STORAGE MEDIUM**

(30) Priority: 09.06.2022 CN 202210646515
(71) Applicant: Hyperbio Biological Technology Co., Ltd, Changsha, Hunan 410000 (CN)
(72) Inventor: HUANG, Qinghong, Changsha, Hunan 410000 (CN); HUANG, Qingxi, Changsha, Hunan 410000 (CN)
(74) Representative: Murgitroyd & Company
(86) International application number: PCT/CN2023/091604
(87) International publication number: WO 2023/236687

(57) **Abstract**

Provided is an exercise electrocardiogram data analysis method, which includes: acquiring and analyzing exercise electrocardiogram data to obtain a high-frequency QRS waveform curve; selecting, from the high-frequency QRS waveform curve, a start point and an end point of an exercise phase as a first reference point and a second reference point respectively; or selecting, from the high-frequency QRS waveform curve, a candidate waveform curve, selecting, from the candidate waveform curve, a first reference point with the maximum root-mean-square voltage and a second reference point with the minimum root-mean-square voltage after the first reference point; or selecting the first reference point with the maximum root-mean-square voltage from the candidate waveform curve, and selecting the end point of the exercise phase as the second reference point; according to the first reference point, the second reference point and the high-frequency QRS waveform curve, determining the area of a waveform descent area; and according to the area of the waveform descent area, determining the attention level.

## Description

### CROSS-REFERENCE TO RELATED APPLICATIONS

The present application claims priority to Chinese patent application No. 2022106465153, titled "EXERCISE ELECTROCARDIOGRAM DATA ANALYSIS METHOD AND APPARATUS, COMPUTER DEVICE AND STORAGE MEDIUM", filed on June 09, 2022, the entire contents of which are incorporated herein by reference.

### TECHNICAL FIELD

The present application relates to an exercise electrocardiogram data analysis method and apparatus, a computer device, and a storage medium.

### BACKGROUND

With the continuous improvement of people's living standards and the continuous increase of work pressure, heart diseases (such as myocardial infarction) are becoming more and more common and younger, and have gradually become one of the major diseases that seriously threaten people's health. Therefore, how to effectively identify a heart health status to achieve preventive monitoring of heart diseases is a problem worthy of attention.

At present, information related to cardiac activity is typically analyzed based on ST-T segment data from an electrocardiogram (ECG) to estimate whether there is myocardial ischemia, so as to achieve the identification of the heart health status. However, the applicant realized that many potential heart problems do not show abnormalities in the ST-T segment data, resulting in reduced accuracy in identifying the heart health status.

### SUMMARY

According to various embodiments disclosed in the present application, an exercise electrocardiogram data analysis method and apparatus, a computer device and a storage medium are provided.

An exercise electrocardiogram data analysis method is provided, which includes:
obtaining exercise electrocardiogram (ECG) data;
analyzing a high-frequency component of a QRS complex in the exercise ECG data to obtain a high-frequency QRS waveform curve;
selecting a first reference point and a second reference point from the high-frequency QRS waveform curve;
determining, according to the first reference point, the second reference point, and the high-frequency QRS waveform curve, a corresponding area of a waveform descent region; and
determining, according to the area of the waveform descent region, an attention level corresponding to the exercise ECG data;
selecting the first reference point and the second reference point from the high-frequency QRS waveform curve includes:
   selecting a start point and an end point of an exercise phase from the high-frequency QRS waveform curve as the first reference point and the second reference point, respectively; or,
   selecting a candidate waveform curve from the high-frequency QRS waveform curve, selecting, from the candidate waveform curve, a point with a maximum RMS voltage as the first reference point, and a point with a minimum RMS voltage after the first reference point as the second reference point; or,
   selecting a candidate waveform curve from the high-frequency QRS waveform curve, selecting a point with the maximum RMS voltage from the candidate waveform curve as the first reference point, and selecting the end point of the exercise phase as the second reference point.

In an embodiment, the area of the waveform descent region includes an absolute descent area, and determining, according to the first reference point, the second reference point, and the high-frequency QRS waveform curve, the area of the corresponding waveform descent region includes:
selecting a curve between the first reference point and the second reference point from the high-frequency QRS waveform curve as a reference waveform curve;
determining, according to the reference waveform curve, a reference amplitude; and
calculating, according to the reference amplitude and the reference waveform curve, the absolute descent area by using a first function.

In an embodiment, the area of the waveform descent region further includes a relative descent area, and determining, according to the first reference point, the second reference point, and the high-frequency QRS waveform curve, the area of the corresponding waveform descent region further includes:
calculating, according to the reference waveform curve, a reference area by using a second function; and
obtaining, according to the absolute descent area and the reference area, the relative descent area.

In an embodiment, the method further includes:
determining, according to the high-frequency QRS waveform curve, a reference index, the reference index including at least one of an amplitude decrease relative value, a lead positive index, a positive position, or a waveform category;
determining, according to the area of the waveform descent region, the attention level corresponding to the exercise ECG data includes:
   determining, according to the area of the waveform descent region and the reference index, the attention level corresponding to the exercise ECG data.

In an embodiment, the method further includes:
obtaining exercise stress test parameters corresponding to the exercise ECG data; and
determining, according to the exercise stress test parameters, a correction coefficient;
determining, according to the area of the waveform descent region, the attention level corresponding to the exercise ECG data includes:
   correcting, according to the correction coefficient, the area of the waveform descent region; and
   determining, according to a corrected area of the waveform descent region, the attention level corresponding to the exercise ECG data.

An exercise electrocardiogram data analysis apparatus is provided, which includes:
an obtaining module configured to obtain exercise electrocardiogram (ECG) data;
an analysis module configured to analyze a high-frequency component of a QRS complex in the exercise ECG data to obtain a high-frequency QRS waveform curve;
a selection module configured to select a first reference point and a second reference point from the high-frequency QRS waveform curve;
an estimation index determination module configured to determine, according to the first reference point, the second reference point, and the high-frequency QRS waveform curve, a corresponding area of a waveform descent region; and
an attention level determination module configured to determine, according to the area of the waveform descent region, an attention level corresponding to the exercise ECG data;
the selection module further configured to: select a start point and an end point of an exercise phase from the high-frequency QRS waveform curve as the first reference point and the second reference point, respectively; or select a candidate waveform curve from the high-frequency QRS waveform curve, select, from the candidate waveform curve, a point with a maximum RMS voltage as the first reference point, and a point with a minimum RMS voltage after the first reference point as the second reference point; or select a candidate waveform curve from the high-frequency QRS waveform curve, select a point with the maximum RMS voltage from the candidate waveform curve as the first reference point, and select the end point of the exercise phase as the second reference point.

A computer device is provided, including a memory and one or more processors. The memory stores computer-readable instructions, and when the computer-readable instructions are executed by the one or more processors, steps of the exercise electrocardiogram data analysis method provided in any one of embodiments of the present application are implemented.

One or more non-transitory storage mediums storing computer-readable instructions are provided. The computer-readable instructions, when executed by one or more processors, cause the one or more processors to perform steps of the exercise electrocardiogram data analysis method provided in any one of embodiments of the present application.

One or more embodiments of the present application will be described in detail below with reference to drawings. Other features, objects and advantages of the present application will become more apparent from the description, drawings, and claims.

### BRIEF DESCRIPTION OF THE DRAWINGS

In order to describe the technical solutions of the embodiments of the present application more clearly, the accompanying drawings required for describing the embodiments will be briefly introduced as follows. Apparently, the accompanying drawings, in the following description, illustrate merely some embodiments of the present application, for a person of ordinary skill in the art, other drawings can also be obtained according to these accompanying drawings without making any creative efforts.
FIG. 1 is a flow diagram illustrating an exercise electrocardiogram data analysis method according to one or more embodiments.
FIG. 2 is a schematic diagram illustrating a method for calculating an area of a waveform descent region based on a high-frequency QRS waveform diagram according to one or more embodiments.
FIG. 3 is a flow diagram illustrating an exercise electrocardiogram data analysis method according to another embodiment.
FIG. 4 is a block diagram illustrating a configuration of an exercise electrocardiogram data analysis apparatus according to one or more embodiments.
FIG. 5 is a diagram illustrating an internal configuration of a computer device according to one or more embodiments.

### DETAILED DESCRIPTION OF THE EMBODIMENTS

In order to make technical solutions and advantages of the present application more clearly understood, the present application will be further described in detail with the accompanying drawings and embodiments. It should be understood that the specific embodiments described herein are only used to explain the present application and not to limit the present application.

An exercise electrocardiogram (ECG) data analysis method provided by the present application may be applied to a terminal, a server, or an interactive system including a terminal and a server, and is implemented through the interaction between the terminal and the server, which is not specifically limited here. The terminal may be, but is not limited to, various personal computers, laptops, smart phones, tablet computers, ECG monitoring devices, and portable wearable devices. The server may be implemented as an independent server or a server cluster composed of a plurality of servers.

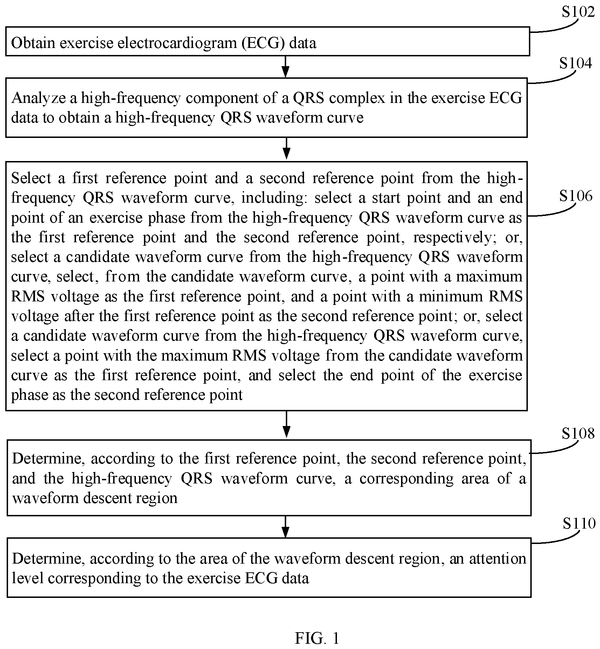

In some embodiments, as shown in FIG. 1, an exercise electrocardiogram data analysis method is provided. Taking the method applied to a server as an example for illustration, the method specifically includes the following steps.

In step S102, exercise ECG data is obtained.

The exercise ECG data refers to ECG data acquired during an exercise stress ECG testing. The exercise stress ECG testing refers to an ECG testing method that increases the cardiac load of a subject through a certain amount of exercise to acquire the ECG data of the subject, and analyzes the heart health status of the subject based on the acquired ECG data. The exercise stress ECG testing is widely applied to the test of heart disease and cardiovascular disease. For example, the exercise ECG data may be used to analyze whether the subject has myocardial ischemia, as well as the severity of the myocardial ischemia.

In some embodiments, a process of the exercise stress ECG testing includes multiple phases, specifically including three phases in sequence: a resting phase, an exercise phase and a recovery phase. The exercise ECG data includes ECG data of respective phases. It can be understood that the division of phases is not limited to thereto, and may be divided according to an actual condition.

In some embodiments, during the exercise stress ECG testing, 10 electrode plates distributed on the chest and limbs of a human body may be used to form 12 ECG leads (such as V1, V2, V3, V4, V5, V6, I, II, III, aVL, aVF and aVR), and 12 sets of ECG data are output correspondingly to obtain the exercise ECG data corresponding to the entire exercise stress ECG testing. It can be understood that the 10 electrode plates are merely used as an example, and are not used to specifically limit the number of electrode plates. The number of electrode plates may be specifically determined dynamically according to an actual need, such as a greater or smaller number of electrode plates.

In step S104, a high-frequency component of a QRS complex in the exercise ECG data is analyzed to obtain a high-frequency QRS waveform curve.

The exercise ECG data includes a plurality of QRS complexes. The QRS complex reflects changes in the depolarization potential and time of left and right ventricles. Each QRS complex is a collection of Q-wave, R-wave, and S-wave in the ECG. A first downward wave in the QRS complex is the Q-wave, a first upward wave in the QRS complex is the R-wave, and a second downward wave in the QRS complex is the S-wave. The high-frequency component of the QRS complex refers to a high-frequency band in the QRS complex with a frequency above 100 Hz, and specifically refers to the high-frequency band in the QRS complex with a frequency within a range of 150 Hz-250 Hz. The high-frequency QRS waveform curve is used to represent a variation trend of a root-mean-square voltage of a high-frequency component of a QRS complex of the subject over time during an entire exercise stress ECG testing (including the resting phase, the exercise phase, and the recovery phase), i.e., the high-frequency QRS waveform curve is used to reflect an energy variation trend during the entire exercise stress ECG testing. The high-frequency QRS waveform curve is presented through a high-frequency QRS waveform diagram. In the high-frequency QRS waveform diagram, the horizontal axis represents time, which corresponds to the test time of the exercise stress ECG testing, i.e., the corresponding signal acquisition time, and the unit is minute (min). The vertical axis represents the root-mean-square voltage (RMS voltage), which may also be understood as intensity or amplitude, and the unit is microvolt (uV).

Specifically, the exercise ECG data includes ECG corresponding to each heartbeat of the subject during the entire exercise stress ECG testing, and the ECG includes a QRS complex. The exercise ECG data is divided into multiple ECG data subsets according to a time sequence and a preset moving step by using a window function, and each ECG data subset includes ECGs corresponding to multiple heartbeats. For each ECG data subset, an alignment processing, an averaging processing, and a bandpass filtering processing are performed sequentially on the ECGs or QRS complexes corresponding to the multiple heartbeats to obtain the corresponding high-frequency QRS complex (the high-frequency band of the QRS complex), and then a RMS processing is performed on the high-frequency QRS complex to obtain the corresponding RMS voltage as the RMS voltage/intensity/amplitude corresponding to the ECG data subset. Further, a curve smoothing processing is performed on the RMS voltage/intensity/amplitude corresponding to each ECG data subset according to the time sequence to obtain the high-frequency QRS waveform curve corresponding to the exercise ECG data.

It can be understood that a window length and a preset moving step of the window function may be customized according to an actual need. For example, the window length is set to 10 seconds, and the preset moving step is set to 10 seconds or one heartbeat cycle, which is not specifically limited here. The heartbeat cycle refers to a time interval between two adjacent heartbeats. The time sequence refers to a sequence of the signal acquisition time, or a sequence of the test time during the exercise stress ECG testing.

In some embodiments, the exercise ECG data includes ECG data corresponding to at least one ECG lead. A high-frequency component analysis of the QRS complex is performed on the ECG data corresponding to each ECG lead to obtain a high-frequency QRS waveform curve corresponding to each ECG lead, so as to calculate an area of a waveform descent region corresponding to each ECG lead according to the high-frequency QRS waveform curve corresponding to the ECG lead, and then determine an attention level corresponding to the exercise ECG data.

In step S106, a first reference point and a second reference point are selected from the high-frequency QRS waveform curve. The step of selecting the first reference point and the second reference point from the high-frequency QRS waveform curve includes: selecting a start point and an end point of an exercise phase from the high-frequency QRS waveform curve as the first reference point and the second reference point, respectively; or selecting a candidate waveform curve from the high-frequency QRS waveform curve, selecting, from the candidate waveform curve, a point with a maximum RMS voltage as the first reference point, and a point with a minimum RMS voltage after the first reference point as the second reference point; or selecting a candidate waveform curve from the high-frequency QRS waveform curve, selecting a point with the maximum RMS voltage from the candidate waveform curve as the first reference point, and selecting the end point of the exercise phase as the second reference point.

The first reference point and the second reference point are used to locate the start and end points of a reference waveform curve from the high-frequency QRS waveform curve, so as to calculate an area of a waveform descent region of the corresponding high-frequency QRS waveform curve according to the reference waveform curve. The time of the first reference point is earlier than/less than the time of the second reference point, i.e., the first reference point appears before the second reference point on the high-frequency QRS waveform curve.

Specifically, the start point and the end point of the exercise phase are determined from the high-frequency QRS waveform curve, the start point of the exercise phase is taken as the first reference point, and the end point of the exercise phase is taken as the second reference point. Alternatively, a curve within a preset time period is selected from the high-frequency QRS waveform curve as the candidate waveform curve, and then the point with the maximum RMS voltage on the candidate waveform curve is selected as the first reference point, and the point with the minimum RMS voltage after the first reference point on the candidate waveform curve is selected as the second reference point. Alternatively, the point with the maximum RMS voltage on the candidate waveform curve is selected as the first reference point, and the end point of the exercise phase is selected as the second reference point. It can be understood that the preset time period may be customized according to an actual need. In an example where the time point in the resting phase 100 seconds away from the start point of the exercise phase is taken as a start point of the preset time period, and the time point in the recovery phase 20 seconds away from the end point of the exercise phase is taken as an end point of the preset time period, and a time range corresponding to the exercise phase in the high-frequency QRS waveform is set to from the 3^{rd} minute to 9^{th} minute as an example, the preset time period may be set to [1 minute 20 seconds, 9 minutes 20 seconds], which is not specifically limited thereto.

In this way, the first reference point and the second reference point with a reference value are selected, so as to determine the area of the waveform descent region with a reference value according to the selected first reference point, the second reference point, and the corresponding high-frequency QRS waveform curve, thereby facilitating improving the accuracy of identifying the heart health status.

In step S108, a corresponding area of a waveform descent region is determined according to the first reference point, the second reference point, and the high-frequency QRS waveform curve.

The area of the waveform descent region includes an absolute descent area and/or a relative descent area, which can be used to estimate myocardial ischemia.

Specifically, a reference amplitude is determined according to the first reference point, the second reference point, and the high-frequency QRS waveform curve, and then a closed region defined by the reference amplitude, the high-frequency QRS waveform curve and the second reference point is determined as the waveform descent region. Further, an area of the waveform descent region is calculated to obtain an absolute descent area, and the absolute descent area is taken as the area of the waveform descent region of the corresponding high-frequency QRS waveform curve. Alternatively, a closed region defined by the first reference point, the high-frequency QRS waveform curve, the second reference point and a horizontal axis may be determined as a reference region. Further, a ratio of the absolute descent area to an area of the reference region is calculated to obtain a relative descent area, and the relative descent area is taken as the area of the waveform descent region of the corresponding high-frequency QRS waveform curve. Alternatively, the absolute descent area and the relative descent area calculated in the above manner are taken as the area of the waveform descent region of the corresponding high-frequency QRS waveform curve. The horizontal axis refers to the horizontal axis of the high-frequency QRS waveform diagram used to display the high-frequency QRS waveform curve, i.e., the reference axis with an RMS voltage/amplitude of zero.

In some embodiments, a closed region defined by the reference amplitude, the first reference point, the second reference point, and the horizontal axis is determined as a reference region. An RMS voltage/amplitude of the first reference point may be determined as the reference amplitude, or a maximum RMS voltage between the first reference point and the second reference point on the high-frequency QRS waveform curve may be determined as the reference amplitude, which is not limited here.

In some embodiments, if the point with the maximum RMS voltage on the candidate waveform curve is taken as the first reference point, the RMS voltage of the first reference point is determined as the reference amplitude. If the start point of the exercise phase is taken as the first reference point, the RMS voltage of the first reference point may be determined as the reference amplitude, or the maximum RMS voltage on the candidate waveform curve may be determined as the reference amplitude.

In step S110, an attention level corresponding to the exercise ECG data is determined according to the area of the waveform descent region.

The attention level is used to represent different levels of attention. The area of the waveform descent region may indicate different degrees of myocardial ischemia and different durations of myocardial ischemia, providing a reference for doctors in diagnosis. For example, the larger the area of the waveform descent region, the higher the degree of myocardial ischemia, and a higher attention level may be set accordingly, so that the doctor can accurately identify the heart health status of the subject based on the attention level, the high-frequency QRS waveform curve, and the area of the waveform descent region, etc., combined with clinical symptoms, and can also give a further diagnosis and treatment suggestion based on the clinical symptoms. It can be understood that myocardial ischemia is a symptom or manifestation rather than a disease. For example, the fact that the subject has myocardial ischemia does not necessarily indicate that the subject has a coronary heart disease, nor does it necessarily indicate that the subject will have a heart disease such as myocardial infarction. Specifically, a heart health level of the subject is determined based on the area of the waveform descent region of the high-frequency QRS waveform curve, and the corresponding attention level is set for the exercise ECG data for the doctor's reference, so that the doctor can refer to the attention level, and give a further detection suggestion or diagnosis and treatment suggestion based on the clinical symptoms.

In some embodiments, after determining the area of the waveform descent region corresponding to at least one ECG lead according to one or more embodiments of the present application, the attention level of the exercise ECG data is determined according to areas of the waveform descent regions corresponding to respective ECG leads. For example, the attention level of the exercise ECG data is determined according to an area threshold interval in which a sum or average value of the areas of the waveform descent regions corresponding to the ECG leads is located, or the attention level of the exercise ECG data is determined according to an area threshold interval in which a maximum area among the areas of the waveform descent regions corresponding to the ECG leads is located, or the attention level of the exercise ECG data is determined according to a distribution of the areas of the waveform descent regions corresponding to the ECG leads in preset area threshold intervals, which is not specifically limited here. The area threshold interval may be customized according to an actual condition.

In the exercise ECG data analysis method as described above, the high-frequency component analysis is performed on the QRS complex in the exercise ECG data to obtain the corresponding high-frequency QRS waveform curve, and the area of the corresponding waveform descent region is determined according to the first reference point and the second reference point selected from the high-frequency QRS waveform curve, and the high-frequency QRS waveform curve. Then the attention level corresponding to the exercise ECG data is determined according to the area of the waveform descent region corresponding to the high-frequency QRS waveform curve for the doctor's reference, so that the doctor can accurately identify the heart health status of the subject combined with clinical symptoms, which can reduce the subjectivity of the doctor's identification of the heart health status, thereby improving the accuracy of identifying the heart health status.

In some embodiments, the area of the waveform descent region includes an absolute descent area. The step S108 includes: selecting a curve between the first reference point and the second reference point from the high-frequency QRS waveform curve as a reference waveform curve; determining a reference amplitude according to the reference waveform curve; and calculating the absolute descent area according to the reference amplitude and the reference waveform curve by using a first function.

The absolute descent area refers to an area of the waveform descent region on the high-frequency QRS waveform curve. The first function is used to calculate an area of a closed region defined by the reference amplitude and the reference waveform curve to obtain the absolute descent area, which is not specifically limited here. If there are multiple closed regions defined by the reference amplitude and the reference waveform curve, only areas of the closed regions below the reference amplitude (the RMS voltage of each point in the closed region is less than or equal to the reference amplitude) are calculated to obtain the absolute descent area.

Specifically, the curve between the first reference point and the second reference point is selected from the high-frequency QRS waveform curve to obtain the reference waveform curve, and the first reference point and the second reference point are taken as the start point and the end point of the reference waveform curve, respectively. A maximum RMS voltage on the reference waveform curve or an RMS voltage at the start point is taken as the reference amplitude, and the closed region defined by the reference amplitude and the reference waveform curve is determined as the waveform descent region of the high-frequency QRS waveform curve. The area of the waveform descent region is calculated by using the first function to obtain the absolute descent area, and the absolute descent area is taken as the area of the waveform descent region of the corresponding high-frequency QRS waveform curve. Taking the area of the waveform descent region including the absolute descent area as an example, the area threshold interval includes an absolute area threshold interval, and four absolute area threshold intervals, from a first absolute area threshold interval to a fourth absolute area threshold interval, are preset, with the priority of attention decreasing sequentially. If the absolute descent area is within the first absolute area threshold interval, the attention level is determined to be a first attention level, and if the absolute descent area is within a second absolute area threshold interval, the attention level is determined to be a second attention level, and so on. The absolute descent area used for comparison with the absolute area threshold interval may be a sum or average value of absolute descent areas corresponding to the ECG leads, or a maximum value of the absolute descent areas corresponding to the ECG leads. If the absolute descent area used for comparison is the average value, then the absolute area threshold intervals are, for example, an interval greater than or equal to 8, an interval greater than or equal to 5 and less than 8, an interval greater than or equal to 3 and less than 5, and an interval less than 3, respectively. The number of absolute area threshold intervals and corresponding interval values are only examples, and are not used for specific limitations.

In the above embodiment, the reference waveform curve is determined based on the high-frequency QRS waveform curve, the first reference point, and the second reference point, and then the absolute descent area of the waveform descent region is calculated based on the reference waveform curve, so as to determine the attention level for the doctor's reference based on the absolute descent area, thereby improving the accuracy of identifying the heart health status.

In some embodiments, the area of the waveform descent region further includes a relative descent area. The step S108 further includes: calculating a reference area according to the reference waveform curve by using a second function; and obtaining the relative descent area according to the absolute descent area and the reference area.

The relative descent area refers to a ratio of the area of the waveform descent region on the high-frequency QRS waveform curve to the corresponding reference area. The second function is used to calculate an area of a closed region defined by the reference waveform curve and the horizontal axis to obtain the reference area, which is not specifically limited here. Specifically, after selecting the reference waveform curve from the high-frequency QRS waveform curve according to the first reference point and the second reference point, a closed region defined by the reference waveform curve and the corresponding reference axis (i.e., the horizontal axis of the high-frequency QRS waveform diagram) with a RMS voltage of zero is determined as a reference region, and an area of the reference region is calculated by using the second function to obtain the reference area. A ratio of the absolute descent area to the reference area is calculated to obtain the relative descent area of the corresponding high-frequency QRS waveform curve, and the corresponding area of the waveform descent region is obtained according to the absolute descent area and the relative descent area of the high-frequency QRS waveform curve.

Taking the area of the waveform descent region further including the relative descent area as an example, the area threshold interval further includes a relative area threshold interval, and four relative area threshold intervals, from a first relative area threshold interval to a fourth relative area threshold interval, are preset, with the priority of attention decreasing sequentially. The corresponding attention level is determined according to various combinations of the absolute area threshold interval in which the absolute descent area is located, and the relative area threshold interval in which the relative descent area is located. For example, if the absolute descent area is within the first absolute area threshold interval, and the relative descent area is within the first relative area threshold interval, the attention level is determined to be a first level. If the absolute descent area is within the first absolute area threshold interval, and the relative descent area is within a second relative area threshold interval, the attention level is determined to be a second level. The examples listed here are not exhaustive. Similarly, the relative descent area used for comparison with the relative area threshold interval may be a sum, average or maximum value of relative descent areas corresponding to the ECG leads. If the relative descent area used for comparison is the average value, then the relative area threshold intervals are, for example, an interval greater than or equal to 50%, an interval greater than or equal to 30% and less than 50%, an interval greater than or equal to 10% and less than 30%, and an interval less than 10%, respectively. The number of relative area threshold intervals and corresponding interval values are only examples, and are not used for specific limitations.

In some embodiments, the area of the waveform descent region is preconfigured with a plurality of area threshold intervals. If the area of the waveform descent region includes the absolute descent area, the respective absolute area threshold intervals are determined as the area threshold intervals. If the area of the waveform descent region includes the absolute descent area and the relative descent area, the area threshold intervals are determined based on a combination of the absolute area threshold intervals and the relative area threshold intervals, and then the attention levels corresponding to the area threshold intervals are determined. For example, a first area threshold interval includes a first absolute area threshold interval and a first relative area threshold interval, and the corresponding attention level is a first attention level. For another example, a second area threshold interval includes the first absolute area threshold interval and a second relative area threshold interval, or a second absolute area threshold interval and the first relative area threshold interval, and the corresponding attention level is a second attention level. By analogy, the plurality of area threshold intervals may be obtained. The examples listed here are not exhaustive. Thus, the corresponding attention level may be obtained based on the area of the waveform descent region of the high-frequency QRS waveform curve and the area threshold interval.

For example, if the area of the waveform descent region is within the first area threshold interval, the attention level is determined to be the first attention level. If the area of the waveform descent region is within the second area threshold interval, the attention level is determined to be the second attention level, and so on. Similarly, the area of the waveform descent region used for comparison with the area threshold interval may be a sum, average or maximum value of the areas of the waveform descent regions corresponding to the ECG leads. Taking the average value as an example, the average value of the areas of the waveform descent regions corresponding to the ECG leads includes the average value of the absolute descent areas corresponding to the ECG leads and the average value of the relative descent areas corresponding to the ECG leads. Correspondingly, if the average value (or the sum/maximum value) of the absolute descent areas corresponding to the ECG leads is within the first absolute area threshold interval in the first area threshold interval, and the average value (or the sum/maximum value) of the relative descent areas corresponding to the ECG leads is within the first relative area threshold interval in the first area threshold interval, then it is determined that the area of the waveform descent region is within the first area threshold interval. Other situations are similar and are not exhausted here.

In the above embodiment, by combining the relative descent area determined by the reference waveform curve and the absolute descent area with the absolute descent area, a more valuable attention level is obtained for the doctor's reference, thereby further improving the accuracy of identifying the heart health status.

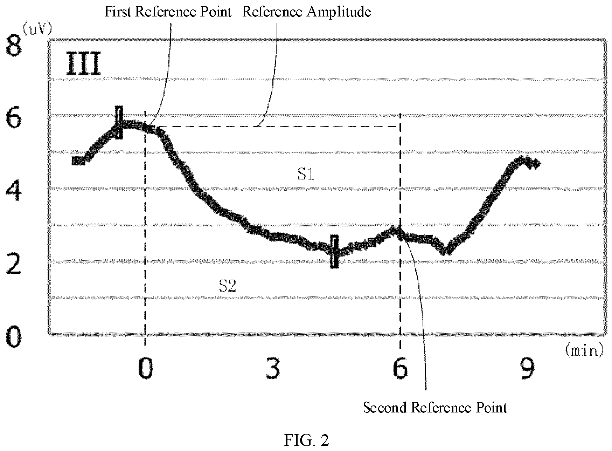

In some embodiments, FIG. 2 is a schematic diagram illustrating a method for calculating a corresponding area of a waveform descent region based on a high-frequency QRS waveform diagram. As shown in FIG. 2, the high-frequency QRS waveform diagram shows a high-frequency QRS waveform curve corresponding to the ECG lead III. The horizontal axis represents time in minute, and the vertical axis represents RMS voltage/amplitude in microvolt. A time range corresponding to the exercise phase is from 0 to the 6^{th} minute on the high-frequency QRS waveform curve. The start point and the end point of the exercise phase are selected as the first reference point and the second reference point, respectively. The RMS voltage of the first reference point is determined as the reference amplitude. The closed region defined by the reference amplitude, the high-frequency QRS waveform curve and the second reference point is determined as the waveform descent region of the high-frequency QRS waveform curve, and then the area S1 of the waveform descent region is determined as the absolute descent area of the high-frequency QRS waveform curve. The closed region defined by the first reference point, the second reference point, the high-frequency QRS waveform curve and the horizontal axis is determined as the reference region, the area S2 of the reference region is determined as the reference area, and the ratio (S 1/S2) of the absolute descent area S1 to the reference area S2 is determined as the relative descent area of the high-frequency QRS waveform curve. The absolute descent area and/or the relative descent area are taken as the area of the waveform descent region of the high-frequency QRS waveform curve.

It can be understood that the high-frequency QRS waveform curve shown in FIG. 2 and the schematic diagram for calculating the area of the waveform descent region are only examples and are not used for specific limitations. For example, the first reference point, the second reference point, the reference amplitude, and the reference region may be selected by referring to the selection method provided in one or more embodiments of the present application, and then the area of the corresponding waveform descent region may be calculated.

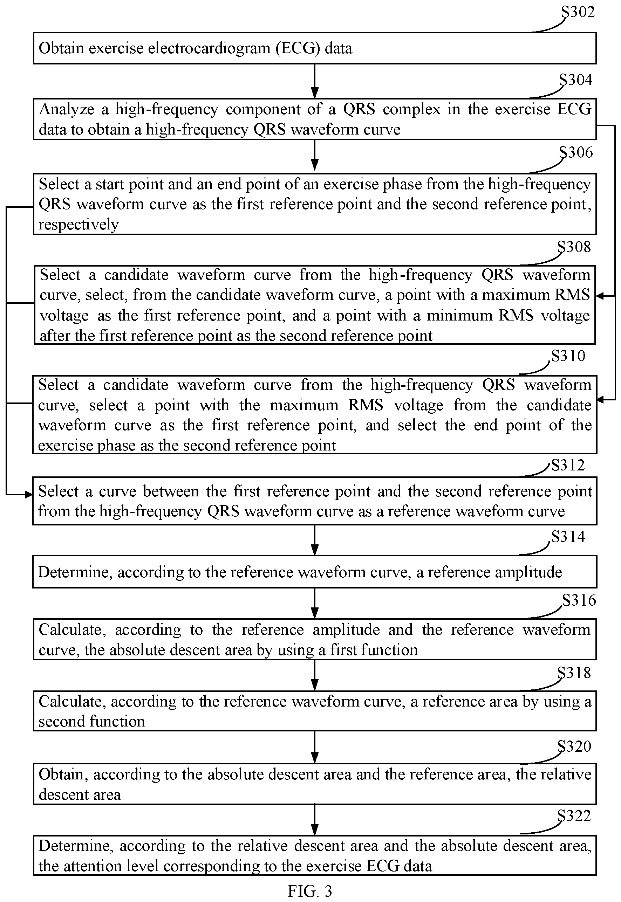

In some embodiments, as shown in FIG. 3, an exercise electrocardiogram data analysis method is provided. The method specifically includes the following steps:

In step S302, exercise ECG data is obtained.

In step S304, a high-frequency component of a QRS complex in the exercise ECG data is analyzed to obtain a high-frequency QRS waveform curve.

In step S306, a start point and an end point of an exercise phase are selected from the high-frequency QRS waveform curve as a first reference point and a second reference point, respectively.

In step S308, a candidate waveform curve is selected from the high-frequency QRS waveform curve, a point with a maximum RMS voltage on the candidate waveform curve is selected as the first reference point, and a point with a minimum RMS voltage on the candidate waveform curve after the first reference point is selected as the second reference point.

In step S310, a candidate waveform curve is selected from the high-frequency QRS waveform curve, a point with a maximum RMS voltage on the candidate waveform curve is selected as the first reference point, and the end point of the exercise phase is selected as the second reference point.

In step S312, a curve between the first reference point and the second reference point is selected from the high-frequency QRS waveform curve as a reference waveform curve.

In step S314, a reference amplitude is determined according to the reference waveform curve.

In step S316, an absolute descent area is calculated according to the reference amplitude and the reference waveform curve by using a first function.

In step S318, a reference area is calculated according to the reference waveform curve by using a second function.

In step S320, a relative descent area is obtained according to the absolute descent area and the reference area.

In step S322, an attention level corresponding to the exercise ECG data is determined according to the relative descent area and the absolute descent area.

In some embodiments, the above exercise ECG data analysis method further includes determining a reference index according to the high-frequency QRS waveform curve. The reference index includes at least one of an amplitude decrease relative value, a lead positive index, a positive position, or a waveform category. The step S110 includes determining the attention level corresponding to the exercise ECG data according to the area of the waveform descent region and the reference index.

The amplitude decrease relative value is used to estimate a change in blood flow of the human heart during exercise, and used to estimate myocardial ischemia. The amplitude decrease relative value may indicate different degrees of myocardial ischemia. For example, the larger the amplitude decrease relative value, the higher the degree of myocardial ischemia. The waveform category is used to indicate a shape category to which a waveform change of the high-frequency QRS waveform curve belongs, and is specifically to indicate an overall variation trend or shape of the high-frequency QRS waveform.

Specifically, the at least one of the amplitude decrease relative value, the lead positive index, the positive position, or the waveform category is determined according to the high-frequency QRS waveform curve. The attention level of the corresponding exercise ECG data is determined based on the area of the waveform descent region, in combination with the at least one of the amplitude decrease relative value, the lead positive index, the positive position, or the waveform category.

In some embodiments, a curve within a preset time period is selected from the high-frequency QRS waveform curve as a candidate waveform curve. A point with the maximum RMS voltage on the candidate waveform curve is selected as a third reference point, and a point with the minimum RMS voltage after the third reference point on the candidate waveform curve is selected as a fourth reference point. The RMS voltage of the fourth reference point is subtracted from the RMS voltage of the third reference point to obtain an amplitude decrease absolute value, and a ratio of the amplitude decrease absolute value to the RMS voltage of the third reference point is determined as the amplitude decrease relative value, so as to comprehensively determine a more valuable attention level for reference in conjunction with the amplitude decrease relative value. It can be understood that the third reference point and the fourth reference point may be appropriately adjusted according to the age, height, weight, and other parameters of the subject.

In some embodiments, a plurality of amplitude threshold intervals are preset for the amplitude decrease relative value, which are used to comprehensively consider the amplitude decrease relative value to determine the attention level. The specific estimate logic is similar to the logic related to the area of the waveform descent region, which will not be repeated here. When comprehensively considering the amplitude decrease relative value to determine the attention level, the corresponding attention level is determined according to various combinations of the amplitude decrease relative value and other reference indexes (such as the area of the waveform descent region). The amplitude threshold interval may be customized according to an actual condition.

Taking the determination of the attention level by combining the amplitude decrease relative value and the area of the waveform descent region as an example, four amplitude threshold intervals, from a first amplitude threshold interval to a fourth amplitude threshold interval, are preset, with the priority of attention decreasing sequentially, which are, for example, an interval greater than or equal to 70%, an interval greater than or equal to 65% and less than 70%, an interval greater than or equal to 55% and less than 65%, and an interval less than 55%, respectively. The corresponding attention level is determined according to various combinations of the area of the waveform descent region and the amplitude decrease relative value. For example, if the area of the waveform descent region is within the first area threshold interval and the amplitude decrease relative value is within the first amplitude threshold interval, then the attention level is determined as the first attention level. If the area of the waveform descent region is within the first area threshold interval and the amplitude decrease relative value is within the second amplitude threshold interval, or if the area of the waveform descent region is within the second area threshold interval and the amplitude decrease relative value is within the first amplitude threshold interval, then the attention level is determined as the second attention level. The examples listed here are not exhaustive. Similarly, the amplitude decrease relative value used for comparison with the amplitude threshold interval may be a sum, average or maximum value of the amplitude decrease relative values corresponding to the ECG leads. The number of amplitude threshold intervals and corresponding interval values are only examples, and are not used for specific limitations.

In some embodiments, after obtaining the amplitude decrease absolute value and the amplitude decrease relative value corresponding to the high-frequency QRS waveform curve, the lead positive index of the corresponding high-frequency QRS waveform curve is determined according to the amplitude decrease relative value and the amplitude decrease absolute value, and is taken as the lead positive index of the corresponding ECG lead, so as to comprehensively determine a more valuable attention level for reference, in conjunction with the lead positive index.

In some embodiments, if the amplitude decrease absolute value and the amplitude decrease relative value of the high-frequency QRS waveform curve each meet a preset condition, the lead positive index indicates that the corresponding ECG lead is positive. The preset condition may be customized according to an actual test situation, and may be adaptively adjusted according to factors such as the age, gender, height, and weight of the subject. For example, the preset condition is that the amplitude decrease absolute value is greater than 1uV and the amplitude decrease relative value is greater than 50%, which is not specifically limited here. If the lead positive index corresponding to the ECG lead indicates positive, the high-frequency QRS waveform curve displayed in the corresponding high-frequency QRS waveform diagram is marked with a warning color. The warning color may be red or yellow, etc., which is not specifically limited here.

In some embodiments, the lead positive index indicates positive, indicating that the change in the blood flow at a myocardial position corresponding to the corresponding ECG lead is abnormal. The more ECG leads that the lead positive index indicates positive, the greater the possibility that there is a problem with the heart. Therefore, by comprehensively considering the lead positive indexes corresponding to the ECG leads, a more valuable attention level may be obtained for the doctor's reference. For example, the attention level is determined according to the areas of the waveform descent regions corresponding to the ECG leads indicated as positive by the lead positive index, such as the sum, average or maximum value of the areas of the waveform descent regions corresponding to the ECG leads indicated as positive by the lead positive index. Alternatively, based on the areas of the waveform descent regions corresponding to the ECG leads (such as the sum, average or maximum value of the areas of the waveform descent regions corresponding to the ECG leads), the attention level is determined in combination with the number of positive leads indicated as positive by the lead positive index. The examples listed here are not exhaustive.

It can be understood that if the attention level is determined in combination with the number of positive leads indicated as positive by the lead positive index, a plurality of quantity threshold intervals may be preset, so as to comprehensively consider the quantity threshold interval in which the number of positive leads is located to determine the attention level. For example, four quantity threshold intervals, from a first quantity threshold interval to a fourth quantity threshold interval, are preset, with the priority of attention decreasing sequentially. These four quantity threshold intervals are, for example, an interval greater than or equal to 8, an interval greater than or equal to 5 and less than 8, an interval greater than or equal to 3 and less than 5, and an interval less than 3, respectively. If the number of positive leads is within the first quantity threshold interval, and the average value (or, sum/maximum value) of the areas of the waveform descent regions corresponding to the ECG leads is within the first area threshold interval, the attention level is determined to be the first attention level. If the number of positive leads is within the first quantity threshold interval, and the average value (or, sum/maximum value) of the areas of the waveform descent regions corresponding to the ECG leads is within the second area threshold interval, or the number of positive leads is within the second quantity threshold interval, and the average value (or, sum/maximum value) of the areas of the waveform descent regions corresponding to the ECG leads is within the first area threshold interval, the attention level is determined to be the second attention level. The examples listed here are not exhaustive. The number of quantity threshold intervals and corresponding interval values are only examples and are not used for specific limitations.

In some embodiments, the areas of the waveform descent regions, the amplitude decrease relative values and the lead positive indexes corresponding to the ECG leads are combined to comprehensively determine a more valuable attention level for the doctor's reference, so as to further improve the accuracy of identifying the heart health status. It can be understood that a corresponding relationship between a combination of the reference indexes and the attention level may be specifically referred to the corresponding relationship provided in one or more embodiments of the present application, and will not be repeated here. For example, the attention level is determined according to the areas (absolute descent area, and/or relative descent area) of the waveform descent regions and the amplitude decrease relative values of the ECG leads indicated as positive by the lead positive index, or the attention level is determined according to the areas of the waveform descent regions and the amplitude decrease relative values of the ECG leads, and the number of positive leads indicated as positive by the lead positive index.

For example, if the average value (or, sum/maximum value) of the areas of the waveform descent regions corresponding to the ECG leads indicated as positive by the lead positive index is within the first area threshold interval, and the average value (or, sum/maximum value) of the amplitude decrease relative values corresponding to the ECG leads indicated as positive by the lead positive index is within the first amplitude threshold interval, then the attention level is determined to be the first attention level. If the average value (or, sum/maximum value) of the areas of the waveform descent regions corresponding to the ECG leads indicated as positive by the lead positive index is within the first area threshold interval, and the average value (or, sum/maximum value) of the amplitude decrease relative values corresponding to the ECG leads indicated as positive by the lead positive index is within the second amplitude threshold interval, then the attention level is determined to be the second attention level. The examples listed here are not exhaustive.

For another example, if the average value (or, sum/maximum value) of the areas of the waveform descent regions corresponding to the ECG leads is within the first area threshold interval, the average value (or, sum/maximum value) of the amplitude decrease relative values corresponding to the ECG leads is within the first amplitude threshold interval, and the number of positive leads indicated as positive by the lead positive index is within the first number threshold interval, then the attention level is determined to be the first attention level. If the average value (or, sum/maximum value) of the areas of the waveform descent regions corresponding to the ECG leads is within the first area threshold interval, the average value (or, sum/maximum value) of the amplitude decrease relative values corresponding to the ECG leads is within the second amplitude threshold interval, and the number of positive leads indicated as positive by the lead positive index is within the first number threshold interval, then the attention level is determined to be the second attention level. The examples listed here are not exhaustive.

In some embodiments, according to a combination of the ECG leads indicated as positive by the lead positive index, a positive position that may be used to estimate a range or region of myocardial ischemia is determined, and then a more valuable attention level for reference may be determined according to the areas of the waveform descent regions and the lead positive indexes of the ECG leads, as well as the positive position. For example, if the ECG leads indicated as positive by the lead positive index include V3, V4, and V5, it may at least be determined that the positive position includes the right ventricle. A plurality of preset positions may be configured for the positive position, and the plurality of preset positions are denoted as a first preset position, a second preset position, and so on, respectively, in the order that the priority of attention decreases sequentially. If the average value (or, sum/maximum value) of the areas of the waveform descent regions corresponding to the ECG leads indicated as positive by the lead positive index is within the first area threshold interval, and the positive position is the first preset position, the attention level is determined to be the first attention level. If the average value (or, sum/maximum value) of the areas of the waveform descent regions corresponding to the ECG leads indicated as positive by the lead positive index is within the first area threshold interval, and the positive position is the second preset position, or the average value (or, sum/maximum value) of the areas of the waveform descent regions corresponding to the ECG leads indicated as positive by the lead positive index is within the second area threshold interval, and the positive position is the first preset position, the attention level is determined to be the second attention level. The examples listed here are not exhaustive.

In some embodiments, by comprehensively considering the areas of the waveform descent regions, the amplitude decrease relative values and the lead positive indexes corresponding to the ECG leads, and the positive position determined by the ECG leads indicated as positive by the lead positive index, a more valuable attention level may be obtained for the doctor's reference. The corresponding relationship between the combination of the reference indexes and the attention level may be specifically referred to the corresponding relationship provided in one or more embodiments of the present application, and will not be repeated here. For example, if the average value (or, sum/maximum value) of the areas of the waveform descent regions corresponding to the ECG leads indicated as positive by the lead positive index is within the first area threshold interval, the average value (or, sum/maximum value) of the amplitude decrease relative values corresponding to the ECG leads indicated as positive by the lead positive index is within the first amplitude threshold interval, and the positive position includes the first preset position, then the attention level is determined to be the first attention level. If the average value (or, sum/maximum value) of the areas of the waveform descent regions corresponding to the ECG leads indicated as positive by the lead positive index is within the first area threshold interval, the average value (or, sum/maximum value) of the amplitude decrease relative values corresponding to the ECG leads indicated as positive by the lead positive index is within the first amplitude threshold interval, and the positive position includes the second preset position, then the attention level is determined to be the second attention level. The examples listed here are not exhaustive.

In some embodiments, the corresponding waveform category is determined according to the high-frequency QRS waveform curve by using a third function, so as to comprehensively determine the attention level corresponding to the exercise ECG data by combining the area of the waveform descent region of the high-frequency QRS waveform curve and the waveform category. Specifically, preset shapes are matched with the high-frequency QRS waveform curve by using the third function, and the waveform category of the high-frequency QRS waveform curve is determined according to the matching degrees. For example, the category of the preset shape with a matching degree greater than or equal to a preset matching degree threshold is determined as the waveform category of the corresponding high-frequency QRS waveform curve. Alternatively, fixed points representing a shape change are selected from the high-frequency QRS waveform curve by using the third function, and a shape category of a graph composed of the fixed points in a time sequence is determined as the waveform category of the corresponding high-frequency QRS waveform curve. The preset matching degree threshold and the preset shape are customized according to an actual condition. For example, the preset matching degree threshold is 80%, and the preset shape includes but is not limited to a W-shape, a V-shape, a small V-shape, a U-shape, a L-shape, an inverted N-shape, and the like.

In some embodiments, the fixed points representing the shape change are located at a peak position and/or a trough position of the high-frequency QRS waveform curve. A difference between the RMS voltages of two adjacent fixed points is calculated in a time sequence by using the third function, and the fixed points whose corresponding difference of the RMS voltages is greater than a preset difference threshold are connected in a time sequence, and a shape category of a graph obtained by the connection processing is determined as the waveform category of the corresponding high-frequency QRS waveform curve. The preset difference threshold may be determined according to a maximum value of differences of the RMS voltages.

In some embodiments, the waveform category may be used to estimate myocardial ischemia. However, limited by the traditional method of determining the waveform category, there is a problem of not being able to accurately determine the waveform category, such as being unable to clearly distinguish between the V-shape and the small V-shape, while the V-shape and the small V-shape can generally estimate different myocardial ischemia conditions. Therefore, by comprehensively considering the area of the waveform descent region and the waveform category, the waveform category can be corrected by the area of the waveform descent region, thereby obtaining a more valuable attention level for reference.

In some embodiments, a plurality of preset categories are configured for the waveform category, so that a more valuable attention level for reference may be determined by comprehensively considering the preset category to which the waveform category belongs based on the area of the waveform descent region. For example, four preset categories, from a first preset category to a fourth preset category, are provided, with the priority of attention decreasing sequentially. For example, the first preset category includes at least one of the U-shape or the L-shape, the second preset category includes the V-shape, the third preset category includes at least one of the W-shape or the small V-shape, and the fourth preset category includes at least one of the inverted N-shape or a flat shape. If the average value (or, sum/maximum value) of the areas of the waveform descent regions of the ECG leads is within the first area threshold interval and the waveform category is the first preset category, the attention level is determined to be the first attention level. If the average value (or, sum/maximum value) of the areas of the waveform descent regions of the ECG leads is within the first area threshold interval and the waveform category is the second preset category, or the average value (or, sum/maximum value) of the areas of the waveform descent regions of the ECG leads is within the second area threshold interval and the waveform category is the first preset category, the attention level is determined to be the second attention level. The examples listed here are not exhaustive. The waveform category being the second preset category indicates that the highest priority of attention of the waveform category corresponding to the ECG leads is the second preset category, meaning that there is no ECG lead whose corresponding waveform category is the first preset category, but there is an ECG lead whose corresponding waveform category is the second preset category.

In some embodiments, based on the area of the waveform descent region and the waveform category, a more valuable attention level may be obtained for the doctor's reference by further comprehensively considering at least one of the amplitude decrease relative value, the lead positive index or the positive position. It can be understood that the corresponding relationship between the combination of the reference indexes and the attention level may be specifically referred to the corresponding relationship provided in one or more embodiments of the present application, and will not be repeated here. For example, if the average value of the areas of the waveform descent regions of the ECG leads is within the first area threshold interval, the average value of the amplitude decrease relative values of the ECG leads is within the first amplitude threshold interval, and the waveform category is the first preset category, then the attention level is determined to be the first attention level. For another example, if the average value of the areas of the waveform descent regions of the ECG leads indicated as positive by the lead positive index is within the first area threshold interval, the average value of the amplitude decrease relative values of the ECG leads indicated as positive by the lead positive index is within the first amplitude threshold interval, and the waveform category is the first preset category, then the attention level is determined to be the first attention level. For another example, based on the above example, if the positive position is the first preset position, the attention level is determined to be the first attention level.

In some embodiments, taking the determination of the attention level by comprehensively considering the area of the waveform descent region, the amplitude decrease relative value, the lead positive index, the positive position and the waveform category as an example, if the average value (or, sum/maximum value) of the areas of the waveform descent regions of the ECG leads indicated as positive by the lead positive index is within the first area threshold interval, the average value (or, sum/maximum value) of the amplitude decrease relative values of the ECG leads indicated as positive by the lead positive index is within the first amplitude threshold interval, the positive position is the first preset position, and the waveform category is the first preset category, then the attention level is determined to be the first attention level. If the average value (or, sum/maximum value) of the areas of the waveform descent regions of the ECG leads indicated as positive by the lead positive index is within the first area threshold interval, the average value (or, sum/maximum value) of the amplitude decrease relative values of the ECG leads indicated as positive by the lead positive index is within the second amplitude threshold interval, the positive position is the first preset position, and the waveform category is the second preset category, then the attention level is determined to be the second attention level. Various combinations of the reference indexes and corresponding attention levels are not listed here.

In the above embodiment, based on the area of the waveform descent region of the high-frequency QRS waveform curve, an attention level that is more closely matched with the myocardial ischemia condition may be obtained for the doctor's reference by comprehensively considering at least one of the reference indexes such as the amplitude decrease relative value, the lead positive index, the positive position and the waveform category, so that the doctor can improve the accuracy of identifying the heart health status based on the more valuable attention level and the clinical symptoms.

In some embodiments, the above exercise ECG data analysis method further includes: obtaining exercise stress test parameters corresponding to the exercise ECG data; and determining a correction coefficient according to the exercise stress test parameters. The step S110 includes: correcting the area of the waveform descent region according to the correction coefficient; and determining the attention level corresponding to the exercise ECG data according to a corrected area of the waveform descent region.

The exercise stress test parameters are test parameters acquired during the exercise stress ECG testing, including but not limited to a stress level, a total metabolic equivalent level, and a ratio of an actual maximum heart rate to a target heart rate. The total metabolic equivalent level refers to a sum of metabolic energy per unit time. The target heart rate is dynamically determined based on the age of the subject, for example, the target heart rate is equal to 220 minus the age of the subject and then the difference is multiplied by 85%.

In some embodiments, the correction coefficient is a function determined by the exercise stress test parameters. The area of the waveform descent region is corrected by multiplying the correction coefficient by the area of the waveform descent region calculated based on the high-frequency QRS waveform curve, and the product is taken as the corrected area of the waveform descent region.

In some embodiments, a weighted summation process is performed on the exercise stress test parameters to obtain a corresponding correction coefficient, and the area of the waveform descent region is corrected according to the correction coefficient. Further, according to the corrected area of the waveform descent region, the attention level of the exercise ECG data is determined according to one or more embodiments of the present application. It can be understood that weights of the exercise stress test parameters may be customized according to an actual need, which are not specifically limited thereto.

In some embodiments, based on the corrected area of the waveform descent region, the attention level of the exercise ECG data is comprehensively determined in combination with at least one of the amplitude decrease relative value, the lead positive index, the positive position, or the waveform category, so as to obtain a more valuable attention level for the doctor's reference. It can be understood that, with reference to the method provided in one or more embodiments of the present application, based on the corrected area of the waveform descent region, the corresponding attention level is determined by comprehensively considering at least one of the reference indexes such as the amplitude decrease relative value, the lead positive index, the positive position, or the waveform category, which will not be repeated here.

In the above embodiment, when determining the attention level of the exercise ECG data, not only the area of the waveform descent region obtained based on the analysis of the exercise ECG data is considered, but also the exercise stress test parameters corresponding to the subject when the exercise ECG data is acquired. Therefore, a more valuable attention level may be obtained for the doctor's reference, thereby improving the accuracy of identifying the heart health status.

It should be understood that although the individual steps in the flow diagrams of FIG. 1 and FIG. 3 are shown sequentially as indicated by arrows, the steps are not necessarily performed sequentially in the order indicated by the arrows. Unless explicitly stated herein, the execution of these steps is not strictly limited in order and these steps can be performed in any other order. Moreover, at least some of the steps in the flow diagrams of FIG. 1 and FIG. 3 may include a plurality of steps or a plurality of phases that are not necessarily performed at the same time, but may be performed at different times. The order in which these steps or phases are performed is not necessarily sequential, and these steps may be performed alternately or alternately with other steps or at least some of the steps or phases in other steps.

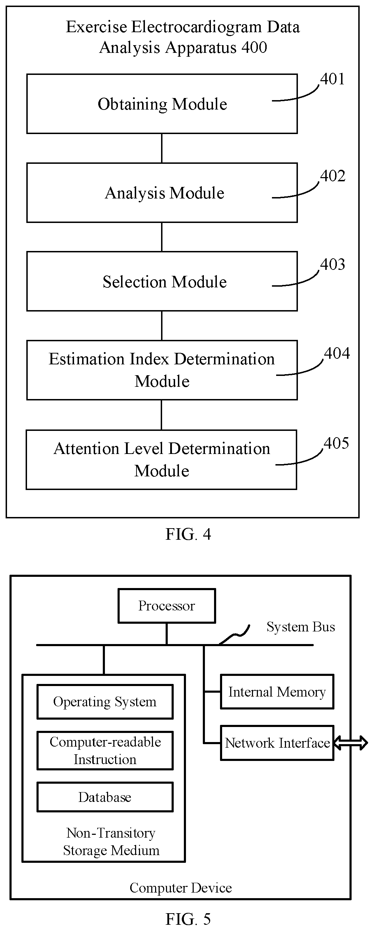

In some embodiments, as shown in FIG. 4, an exercise electrocardiogram data analysis apparatus 400 is provided. The apparatus 400 includes an obtaining module 401, an analysis module 402, a selection module 403, an estimation index determination module 404, and an attention level determination module 405. The obtaining module 401 is configured to obtain exercise electrocardiogram (ECG) data. The analysis module 402 is configured to analyze a high-frequency component of a QRS complex in the exercise ECG data to obtain a high-frequency QRS waveform curve. The selection module 403 is configured to select a first reference point and a second reference point from the high-frequency QRS waveform curve. The estimation index determination module 404 is configured to determine, according to the first reference point, the second reference point, and the high-frequency QRS waveform curve, a corresponding area of a waveform descent region. The attention level determination module 405 is configured to determine, according to the area of the waveform descent region, an attention level corresponding to the exercise ECG data. The selection module is further configured to: select a start point and an end point of an exercise phase from the high-frequency QRS waveform curve as the first reference point and the second reference point, respectively; or select a candidate waveform curve from the high-frequency QRS waveform curve, and select, from the candidate waveform curve, a point with a maximum RMS voltage as the first reference point and a point with a minimum RMS voltage after the first reference point as the second reference point; or select a candidate waveform curve from the high-frequency QRS waveform curve, select a point with the maximum RMS voltage on the candidate waveform curve as the first reference point, and select the end point of the exercise phase as the second reference point.

In some embodiments, the area of the waveform descent region includes an absolute descent area. The estimation index determination module 404 is further configured to select a curve between the first reference point and the second reference point from the high-frequency QRS waveform curve as a reference waveform curve; determine, according to the reference waveform curve, a reference amplitude; and calculate, according to the reference amplitude and the reference waveform curve, the absolute descent area by using a first function.

In some embodiments, the area of the waveform descent region further includes a relative descent area. The estimation index determination module 404 is further configured to calculate, according to the reference waveform curve, a reference area by using a second function; and obtain, according to the absolute descent area and the reference area, the relative descent area.

In some embodiments, the estimation index determination module 404 is further configured to determine, according to the high-frequency QRS waveform curve, a reference index. The reference index includes at least one of an amplitude decrease relative value, a lead positive index, a positive position, or a waveform category. The attention level determination module 405 is further configured to determine, according to the area of the waveform descent region and the reference index, the attention level corresponding to the exercise ECG data.

In some embodiments, the obtaining module 401 is further configured to obtain exercise stress test parameters corresponding to the exercise ECG data. The estimation index determination module 404 is further configured to determine, according to the exercise stress test parameters, a correction coefficient. The attention level determination module 405 is further configured to: correct, according to the correction coefficient, the area of the waveform descent region; and determine, according to a corrected area of the waveform descent region, the attention level corresponding to the exercise ECG data.

For the specific limitations of the exercise electrocardiogram data analysis apparatus, may refer to the limitations of the exercise electrocardiogram data analysis method as described above, which will not be repeated here. The individual modules in the above exercise electrocardiogram data analysis apparatus can be implemented in whole or in part by software, hardware and combinations thereof. Each of the above modules may be embedded in hardware form or independent of a processor in a computer device, or may be stored in software form on a memory in the computer device so that the processor can be called to perform the operations corresponding to each of the above modules.

In some embodiments, a computer device is provided, which may be a server. A diagram illustrating an internal configuration of the computer device may be shown in FIG. 5. The computer device includes a processor, a memory, and a network interface connected via a system bus. The processor of the computer device is configured to provide computing and control capabilities. The memory of the computer device includes a non-transitory storage medium and an internal memory. The non-transitory storage medium stores an operating system, computer-readable instructions, and a database. The internal memory provides an environment for the operation of the operating system and the computer-readable instructions in the non-transitory storage medium. The database of the computer device is used to store exercise electrocardiogram data. The network interface of the computer device is used to communicate with an external terminal through a network connection. When the computer-readable instructions are executed by the processor, the exercise electrocardiogram data analysis method is implemented.

It should be understood by a person of ordinary skill in the art that the configuration illustrated in FIG. 5 is only a block diagram of part of the configuration related to the solution of the present application, and does not constitute a limitation on the computer device to which the solution of the present application is applied. A specific computer device may include more or less components than those shown in the figure, or may combine some components, or may have a different arrangement of components.

In some embodiments, a computer device is further provided, including a memory and one or more processors. The memory stores computer-readable instructions, and when the computer-readable instructions are executed by the processor, steps of the exercise electrocardiogram data analysis method provided in any one of embodiments of the present application are implemented.

In some embodiments, one or more non-transitory storage mediums store computer-readable instructions. The computer-readable instructions, when executed by one or more processors, cause the one or more processors to perform steps of the exercise electrocardiogram data analysis method provided in any one of embodiments of the present application.

A person of ordinary skill in the art may understand that implementation of all or part of the processes in the methods of the above embodiments may be completed by instructing the relevant hardware through computer-readable instructions. The computer-readable instructions may be stored in a non-transitory computer-readable storage medium. When the computer-readable instructions are executed, it may include the processes of the embodiments of the above methods. Any reference to memory, database or other medium used of the embodiments provided in the present application may include at least one of a non-transitory or a transitory memory. The non-transitory memory may include a read-only memory (ROM), a magnetic tape, a floppy disk, a flash memory, an optical memory, etc. The transitory memory may include a random-access memory (RAM) or an external cache memory, etc. As an illustration rather than a limitation, the random-access memory may be in various forms, such as a static random-access memory (SRAM) or a dynamic random-access memory (DRAM), etc.

The technical features in the above embodiments may be combined arbitrarily. For concise description, not all possible combinations of the technical features in the above embodiments are described. However, provided that they do not conflict with each other, all combinations of the technical features are to be considered to be within the scope described in this specification.

The above-mentioned embodiments only describe several implementations of the present application, and their description is specific and detailed, but should not be understood as a limitation on the patent scope of the present application. It should be noted that, for a person of ordinary skill in the art may further make variations and improvements without departing from the conception of the present application, and these all fall within the protection scope of the present application. Therefore, the protection scope of the present application should be subject to the appended claims.

## Claims

1. An exercise electrocardiogram data analysis method, comprising:
obtaining exercise electrocardiogram (ECG) data;
analyzing a high-frequency component of a QRS complex in the exercise ECG data to obtain a high-frequency QRS waveform curve, the high-frequency QRS waveform curve representing a variation trend of a root-mean-square (RMS) voltage of a high-frequency component of a QRS complex of a subject over time during an entire exercise stress ECG testing;
selecting a first reference point and a second reference point from the high-frequency QRS waveform curve;
determining, according to the first reference point, the second reference point, and the high-frequency QRS waveform curve, a corresponding area of a waveform descent region; and
determining, according to the area of the waveform descent region, an attention level corresponding to the exercise ECG data;
wherein selecting the first reference point and the second reference point from the high-frequency QRS waveform curve comprises:
selecting a start point and an end point of an exercise phase from the high-frequency QRS waveform curve as the first reference point and the second reference point, respectively; or,
selecting a candidate waveform curve from the high-frequency QRS waveform curve, selecting, from the candidate waveform curve, a point with a maximum RMS voltage as the first reference point, and a point with a minimum RMS voltage after the first reference point as the second reference point; or,
selecting a candidate waveform curve from the high-frequency QRS waveform curve, selecting a point with the maximum RMS voltage from the candidate waveform curve as the first reference point, and selecting the end point of the exercise phase as the second reference point.

2. The method according to claim 1, wherein the area of the waveform descent region comprises an absolute descent area, and determining, according to the first reference point, the second reference point, and the high-frequency QRS waveform curve, the area of the corresponding waveform descent region comprises:
selecting a curve between the first reference point and the second reference point from the high-frequency QRS waveform curve as a reference waveform curve;
determining, according to the reference waveform curve, a reference amplitude; and
calculating, according to the reference amplitude and the reference waveform curve, the absolute descent area by using a first function.

3. The method according to claim 2, wherein the area of the waveform descent region further comprises a relative descent area, and determining, according to the first reference point, the second reference point, and the high-frequency QRS waveform curve, the area of the corresponding waveform descent region further comprises:
calculating, according to the reference waveform curve, a reference area by using a second function; and
obtaining, according to the absolute descent area and the reference area, the relative descent area.

4. The method according to any one of claims 1 to 3, further comprising:
determining, according to the high-frequency QRS waveform curve, a reference index, the reference index comprising at least one of an amplitude decrease relative value, a lead positive index, a positive position, or a waveform category;
wherein determining, according to the area of the waveform descent region, the attention level corresponding to the exercise ECG data comprises:
determining, according to the area of the waveform descent region and the reference index, the attention level corresponding to the exercise ECG data.

5. The method according to any one of claims 1 to 3, further comprising:
obtaining exercise stress test parameters corresponding to the exercise ECG data; and
determining, according to the exercise stress test parameters, a correction coefficient;
wherein determining, according to the area of the waveform descent region, the attention level corresponding to the exercise ECG data comprises:
correcting, according to the correction coefficient, the area of the waveform descent region; and
determining, according to a corrected area of the waveform descent region, the attention level corresponding to the exercise ECG data.

6. An exercise electrocardiogram data analysis apparatus, comprising:
an obtaining module configured to obtain exercise electrocardiogram (ECG) data;
an analysis module configured to analyze a high-frequency component of a QRS complex in the exercise ECG data to obtain a high-frequency QRS waveform curve, the high-frequency QRS waveform curve representing a variation trend of a root-mean-square (RMS) voltage of a high-frequency component of a QRS complex of a subject over time during an entire exercise stress ECG testing;
a selection module configured to select a first reference point and a second reference point from the high-frequency QRS waveform curve;
an estimation index determination module configured to determine, according to the first reference point, the second reference point, and the high-frequency QRS waveform curve, a corresponding area of a waveform descent region; and
an attention level determination module configured to determine, according to the area of the waveform descent region, an attention level corresponding to the exercise ECG data;
wherein the selection module is further configured to: select a start point and an end point of an exercise phase from the high-frequency QRS waveform curve as the first reference point and the second reference point, respectively; or select a candidate waveform curve from the high-frequency QRS waveform curve, select, from the candidate waveform curve, a point with a maximum RMS voltage as the first reference point, and a point with a minimum RMS voltage after the first reference point as the second reference point; or select a candidate waveform curve from the high-frequency QRS waveform curve, select a point with the maximum RMS voltage from the candidate waveform curve as the first reference point, and select the end point of the exercise phase as the second reference point.

7. The apparatus according to claim 6, wherein the area of the waveform descent region comprises an absolute descent area, and the estimation index determination module is further configured to select a curve between the first reference point and the second reference point from the high-frequency QRS waveform curve as a reference waveform curve; determine, according to the reference waveform curve, a reference amplitude; and calculate, according to the reference amplitude and the reference waveform curve, the absolute descent area by using a first function.

8. The apparatus according to claim 7, wherein the area of the waveform descent region further comprises a relative descent area, and the estimation index determination module is further configured to calculate, according to the reference waveform curve, a reference area by using a second function; and obtain, according to the absolute descent area and the reference area, the relative descent area.

9. The apparatus according to any one of claims 6 to 8, wherein the estimation index determination module is further configured to determine, according to the high-frequency QRS waveform curve, a reference index, the reference index comprises at least one of an amplitude decrease relative value, a lead positive index, a positive position, or a waveform category, and the attention level determination module is further configured to determine, according to the area of the waveform descent region and the reference index, the attention level corresponding to the exercise ECG data.

10. The apparatus according to any one of claims 6 to 8, wherein the obtaining module is further configured to obtain exercise stress test parameters corresponding to the exercise ECG data, the estimation index determination module is further configured to determine, according to the exercise stress test parameters, a correction coefficient, and the attention level determination module is further configured to: correct, according to the correction coefficient, the area of the waveform descent region; and determine, according to a corrected area of the waveform descent region, the attention level corresponding to the exercise ECG data.

11. A computer device comprising a memory and one or more processors, wherein the memory stores computer-readable instructions, and the computer-readable instructions, when executed by the one or more processors, cause the one or more processors to perform:
obtaining exercise electrocardiogram (ECG) data;
analyzing a high-frequency component of a QRS complex in the exercise ECG data to obtain a high-frequency QRS waveform curve, the high-frequency QRS waveform curve representing a variation trend of a root-mean-square (RMS) voltage of a high-frequency component of a QRS complex of a subject over time during an entire exercise stress ECG testing;
selecting a first reference point and a second reference point from the high-frequency QRS waveform curve;
determining, according to the first reference point, the second reference point, and the high-frequency QRS waveform curve, a corresponding area of a waveform descent region; and
determining, according to the area of the waveform descent region, an attention level corresponding to the exercise ECG data;
wherein the one or more processors, when executing the computer-readable instructions, further perform:
selecting a start point and an end point of an exercise phase from the high-frequency QRS waveform curve as the first reference point and the second reference point, respectively; or,
selecting a candidate waveform curve from the high-frequency QRS waveform curve, selecting, from the candidate waveform curve, a point with a maximum RMS voltage as the first reference point, and a point with a minimum RMS voltage after the first reference point as the second reference point; or,
selecting a candidate waveform curve from the high-frequency QRS waveform curve, selecting a point with the maximum RMS voltage from the candidate waveform curve as the first reference point, and selecting the end point of the exercise phase as the second reference point.

12. The computer device according to claim 11, wherein the area of the waveform descent region comprises an absolute descent area, and the one or more processors, when executing the computer-readable instructions, further perform:
selecting a curve between the first reference point and the second reference point from the high-frequency QRS waveform curve as a reference waveform curve;
determining, according to the reference waveform curve, a reference amplitude; and
calculating, according to the reference amplitude and the reference waveform curve, the absolute descent area by using a first function.

13. The computer device according to claim 12, wherein the area of the waveform descent region further comprises a relative descent area, and the one or more processors, when executing the computer-readable instructions, further perform:
calculating, according to the reference waveform curve, a reference area by using a second function; and
obtaining, according to the absolute descent area and the reference area, the relative descent area.

14. The computer device according to any one of claims 11 to 13, wherein the one or more processors, when executing the computer-readable instructions, further perform:
determining, according to the high-frequency QRS waveform curve, a reference index; the reference index comprising at least one of an amplitude decrease relative value, a lead positive index, a positive position, or a waveform category; and
wherein the one or more processors, when executing the computer-readable instructions, further perform:
determining, according to the area of the waveform descent region and the reference index, the attention level corresponding to the exercise ECG data.

15. The computer device according to any one of claims 11 to 13, wherein the one or more processors, when executing the computer-readable instructions, further perform:
obtaining exercise stress test parameters corresponding to the exercise ECG data; and
determining, according to the exercise stress test parameters, a correction coefficient; and
wherein the one or more processors, when executing the computer-readable instructions, further perform:
correcting, according to the correction coefficient, the area of the waveform descent region; and
determining, according to a corrected area of the waveform descent region, the attention level corresponding to the exercise ECG data.

16. One or more non-transitory computer-readable storage mediums storing computer-readable instructions, wherein the computer-readable instructions, when executed by one or more processors, cause the one or more processors to perform:
obtaining exercise electrocardiogram (ECG) data;
analyzing a high-frequency component of a QRS complex in the exercise ECG data to obtain a high-frequency QRS waveform curve, the high-frequency QRS waveform curve representing a variation trend of a root-mean-square (RMS) voltage of a high-frequency component of a QRS complex of a subject over time during an entire exercise stress ECG testing;
selecting a first reference point and a second reference point from the high-frequency QRS waveform curve;
determining, according to the first reference point, the second reference point, and the high-frequency QRS waveform curve, a corresponding area of a waveform descent region; and
determining, according to the area of the waveform descent region, an attention level corresponding to the exercise ECG data; and
wherein the one or more processors, when executing the computer-readable instructions, further perform:
selecting a start point and an end point of an exercise phase from the high-frequency QRS waveform curve as the first reference point and the second reference point, respectively; or,
selecting a candidate waveform curve from the high-frequency QRS waveform curve, selecting, from the candidate waveform curve, a point with a maximum RMS voltage as the first reference point, and a point with a minimum RMS voltage after the first reference point as the second reference point; or,
selecting a candidate waveform curve from the high-frequency QRS waveform curve, selecting a point with the maximum RMS voltage from the candidate waveform curve as the first reference point, and selecting the end point of the exercise phase as the second reference point.

17. The storage medium according to claim 16, wherein the area of the waveform descent region comprises an absolute descent area, and the one or more processors, when executing the computer-readable instructions, further perform:
selecting a curve between the first reference point and the second reference point from the high-frequency QRS waveform curve as a reference waveform curve;
determining, according to the reference waveform curve, a reference amplitude; and
calculating, according to the reference amplitude and the reference waveform curve, the absolute descent area by using a first function.

18. The storage medium according to claim 17, wherein the area of the waveform descent region further comprises a relative descent area, and the one or more processors, when executing the computer-readable instructions, further perform:
calculating, according to the reference waveform curve, a reference area by using a second function; and
obtaining, according to the absolute descent area and the reference area, the relative descent area.

19. The storage medium according to any one of claims 16 to 18, wherein the one or more processors, when executing the computer-readable instructions, further perform:
determining, according to the high-frequency QRS waveform curve, a reference index; the reference index comprising at least one of an amplitude decrease relative value, a lead positive index, a positive position, or a waveform category; and
wherein the one or more processors, when executing the computer-readable instructions, further perform:
determining, according to the area of the waveform descent region and the reference index, the attention level corresponding to the exercise ECG data.

20. The storage medium according to any one of claims 16 to 18, wherein the one or more processors, when executing the computer-readable instructions, further perform:
obtaining exercise stress test parameters corresponding to the exercise ECG data; and
determining, according to the exercise stress test parameters, a correction coefficient; and
wherein the one or more processors, when executing the computer-readable instructions, further perform:
correcting, according to the correction coefficient, the area of the waveform descent region; and
determining, according to a corrected area of the waveform descent region, the attention level corresponding to the exercise ECG data.
